# EUROPÄISCHE PATENTANMELDUNG

(11) **EP 2 386 265 A1**
(43) Veröffentlichungstag der Anmeldung: **16.11.2011**
(21) Anmeldenummer: 11001108.7
(22) Anmeldetag: 11.02.2011
(51) Int. Cl.: A61F 2/00, A61F 2/18, A61L 2/26

(54) **Transportvorrichtung zur Fixierung von thermisch vorbehandelten Mittelohrprothesen aus Memory-Metall mit Platzhalter-Pins**

(30) Priorität: 12.05.2010 DE 202010006737 U; 24.06.2010 DE 102010024899
(71) Anmelder: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus

(57) **Zusammenfassung**

Eine Transportvorrichtung (1) mit steril verpackter Gehörknöchelchenprothese (2), bei der zumindest Teile aus einem Material mit Formgedächtnis hergestellt sind, welche thermisch vorbehandelt und bei der Implantation einer weiteren Form verändernden thermischen Behandlung unterzogen werden, ist dadurch gekennzeichnet, dass sie eine Halterungsvorrichtung umfasst, die zur Aufnahme und gegen Herausfallen gesicherten Halterung der Prothese während des Transports zum Operateur eingerichtet ist, und dass ein oder mehrere in die Halterungsvorrichtung einsteckbare oder integrierte Platzhalter-Pins (9a) vorgesehen sind, die so geformt sind, dass sie die thermisch vorbehandelten, aus Material mit Formgedächtnis bestehenden Teile der Gehörknöchelchenprothese während des Transports in der gewünschten Geometrie derart fixieren, dass auch bei einer Erwärmung während des Transports Formveränderungen der Gehörknöchelchenprothese verhindert werden. Diese Transportvorrichtung wirkt auch ohne Verwendung eines zusätzlichen Kühlsystems gegen thermische Deformationen und bietet darüber hinaus auch noch einen wirksamen Schutz gegen mechanische Verformungen der Prothese.

## Beschreibung

### Beschreibung:

Die Erfindung betrifft eine Transportvorrichtung mit steril verpackter Gehörknöchelchenprothese zur Implantation ins Mittelohr, wobei die Gehörknöchelchenprothese mindestens teilweise die menschliche Gehörknöchelchenkette ersetzen oder überbrücken kann, und wobei zumindest Teile der Gehörknöchelchenprothese aus einem Material mit Formgedächtnis (= memory effect) hergestellt sind, welche thermisch vorbehandelt und bei der Implantation der Gehörknöchelchenprothese im Mittelohr einer weiteren Form verändernden thermischen Behandlung unterzogen werden.

Eine derartige Transportvorrichtung für Gehörknöchelchenprothesen ist bekannt aus der DE 10 2009 006 047 B3.

Gehörknöchelchenprothesen werden verwendet, um bei ganz oder teilweise fehlenden oder geschädigten Gehörknöchelchen des menschlichen Mittelohrs den Schall bzw. das Schallsignal vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Drei besonders häufig verwendete Arten von Gehörknöchelchenprothesen sind die Steigbügel-Prothesen, die Partial-Prothesen und die Total-Prothesen. Steigbügel-Prothesen (= Stapes-Prothesen) werden am Amboss fixiert und ragen über einen Stempel (= Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Da Gehörknöchelchenprothesen naturgemäß mit ihren Außenabmessungen im Bereich von wenigen Millimetern liegen und in ihrem funktionellen Feinaufbau sehr fragil sind, müssen gattungsgemäße Transportvorrichtungen, mit denen derartig zerbrechliche Prothesen vom Hersteller zum Operateur transportiert werden, nicht nur den hygienischen Anforderungen an eine sterile Verpackung genügen, sondern auch sicherstellen, dass die jeweilige Prothese unbeschädigt und undeformiert ankommt.

Denkbar für diesen Zweck ist der Einsatz von Frästeil-Halterungen oder Spritzgussteilen aus Vollmaterial, die Aushöhlungen von geeigneter Form und Größe enthalten, um die Gehörknöchelchenprothese für den Transport aufzunehmen, jedoch bei der Herstellung relativ hohe Kosten verursachen.

Die US-A 4,288,066 beschreibt beispielsweise einen Behälter für eine Mittelohrprothese, der sowohl zum Transport der Prothese zum Operateur als auch zu deren mechanischer Bearbeitung durch den Operateur geeignet sein soll. Diese bekannte Transportvorrichtung besteht aus einem - relativ dicken - Spritzguss-Behälter aus Kunststoff mit geeigneter Ausnehmung zum Einlegen der Prothese sowie einem mittels einer Lasche verklappbar an dem Behälter anhängenden Deckel zum Verschließen des Behälters. Die Ausnehmung ist so gestaltet, dass die Mittelohrprothese vor der Implantation, aber noch unmittelbar vor ihrer Entnahme aus dem Behälter auf ein gewünschtes Längenmaß gekürzt werden kann. Nachteilig bei diesem bekannten Behälter sind einmal seine - im Verhältnis zur Prothesengröße - relativ sperrigen Abmaße, sein komplizierter innerer Aufbau und die daraus resultierenden Kosten für das entsprechende Fertigungswerkzeug sowie die Festlegung auf jeweils genau eine bestimmte Prothesenform und Prothesengröße durch die vom Spritzgusswerkzeug für den Behälter vorgegebene Geometrie.

In der eingangs zitierten DE 10 2009 006 047 B3 wird darüber hinaus eine gattungsgemäße Transportvorrichtung mit steril verpackter Gehörknöchelchenprothese beschrieben, bei welcher zumindest Teile aus einem Material mit Formgedächtnis hergestellt sind, die bei der Herstellung thermisch vorbehandelt und - nach dem Transport - bei der Implantation der Gehörknöchelchenprothese im Mittelohr einer weiteren Form verändernden thermischen Behandlung unterzogen werden. Derartige Prothesen, bei denen mindestens ein Befestigungselement aus Nitinol besteht und intra-operativ im Mittelohr durch thermische Behandlung in seine endgültige Form gebracht wird, sind beispielsweise vorbekannt aus der WO 02/069850 A1 oder aus der US 6,554,861 B2.

Hier ergibt sich gegenüber dem Teil des Standes der Technik, der lediglich "normale" Mittelohrprothesen ohne Verwendung von Memory-Material umfasst, die zusätzliche Problematik, dass die Transportvorrichtung auf ihrer Reise vom Hersteller zum Operateur eventuell ungewollt einer schädlichen Wärmeeinstrahlung ausgesetzt wird, etwa dann, wenn der Transportcontainer in der Sonne steht. Dadurch kann es im Inneren der Transportvorrichtung zu einer Erhitzung kommen, welche auch die zu transportierende Gehörknöchelchenprothese erfasst und diese möglicherweise auf Temperaturen aufheizt, die vergleichbar sind mit denen der prä- und intraoperativen Bearbeitungstemperaturen. Auf diese Weise kann es zu unkontrollierten, thermisch bedingten Deformationen der empfindlichen Gehörknöchelchenprothese kommen, welche letztere im schlimmsten Fall für den weiteren Gebrauch völlig ungeeignet machen und damit - im wirtschaftlichen Sinne - zerstören.

Zum Schutz der besonders empfindlichen, thermisch vorbehandelten Abschnitte der Gehörknöchelchenprothese während des Transports sollten daher zusätzliche Maßnahmen zum Schutz vor thermisch bedingter Deformation ergriffen werden.

Diese Maßnahmen könnten prinzipiell darin bestehen, die Transportvorrichtung mit einem Thermostatensystem, genauer gesagt mit einem Kryostaten auszurüsten, was jedoch mit ganz erheblichen Mehrkosten verbunden wäre.

Aufgabe der vorliegenden Erfindung ist es demgegenüber, mit möglichst einfachen technischen Mitteln unaufwändig und kostengünstig eine Transportvorrichtung für Gehörknöchelchenprothesen mit thermisch vorbehandelten Teilen aus Memory-Material der eingangs beschriebenen Art bereit zu stellen, die - ohne Verwendung eines zusätzlichen Kühlsystems - eine thermische Deformation der Gehörknöchelchenprothese während des Transport selbst im Falle einer ungewollten Erwärmung sicher verhindert und darüber hinaus auch noch einen Schutz gegen mechanische Verformungen der Prothese bietet.

Erfindungsgemäß wird diese Aufgabe auf ebenso überraschend einfache wie wirkungsvolle Weise dadurch gelöst, dass die Transportvorrichtung eine Halterungsvorrichtung umfasst, die zur Aufnahme und gegen Herausfallen gesicherten Halterung der Gehörknöchelchenprothese während des Transports der Prothese zum Operateur eingerichtet ist, und dass ein oder mehrere in die Halterungsvorrichtung einsteckbare oder in der Halterungsvorrichtung integrierte Platzhalter-Pins vorgesehen sind, die so geformt sind, dass sie die thermisch vorbehandelten, aus Material mit Formgedächtnis bestehenden Teile der Gehörknöchelchenprothese während des Transports zum Operateur in der gewünschten Geometrie derart fixieren, dass auch bei einer eventuellen Erwärmung während des Transports ungewünschte Formveränderungen der Gehörknöchelchenprothese verhindert werden.

Die Platzhalter-Pins können zylindrische oder konische Form haben und werden - ebenso wie die Halterungsvorrichtung - bevorzugt aus Metall gefertigt sein, um durch effektive Wärmeableitung einen zusätzlichen Schutz gegen ungewollte thermische Deformationen der zu transportierenden Gehörknöchelchenprothese zu gewährleisten.

Die erfindungsgemäße Transportvorrichtung lässt sich problemlos kompakt in Außenabmessungen herstellen, die kaum größer sind als die der aufzunehmenden Gehörknöchelchenprothese, wobei das Gewicht der Vorrichtung - abhängig von der Wahl des Ausgangsmaterials - ebenfalls in die Größenordnung des Prothesengewichts kommt.

Besonders bevorzugt ist eine Klasse von Ausführungsformen der erfindungsgemäßen Transportvorrichtung, bei denen die Halterungsvorrichtung für die Aufnahme und Halterung der Gehörknöchelchenprothese eine flache Grundplatte von geringer Dicke umfasst.

Eine sehr einfach und kostengünstig herzustellende Weiterbildung dieser Ausführungsformen zeichnet sich dadurch aus, dass die Grundplatte aus dünnem Metallblech - in der Regel in der Größenordnung von etwa einem halben Millimeter - gefertigt ist. Dies hat - wie bereits oben erwähnt - auch noch zusätzliche Vorteile aufgrund der guten Wärmeleitfähigkeit von Metallen.

Die erfindungsgemäße Transportvorrichtung wird besonders bevorzugt durch Bearbeitung der Grundplatte mittels Laserbehandlung und/oder Anodisierung mit hoher Genauigkeit der zu erzeugenden feinen Strukturen hergestellt.

Einen beträchtlichen Zusatznutzen bieten Ausführungsformen der erfindungsgemäßen Transportvorrichtung, bei welchen die Grundplatte als Arbeitsplatte zur Bearbeitung der Gehörknöchelchenprothese vor der Implantation ins Mittelohr ausgestaltet ist.

Insbesondere kann bei bevorzugten Weiterbildungen dieser Ausführungsformen die Gehörknöchelchenprothese längenvariabel aufgebaut und die Grundplatte als Arbeitsplatte zur Einstellung einer individuell gewünschten Länge der Gehörknöchelchenprothese ausgestaltet sein.

Ganz besonders bevorzugt sind auch Weiterbildungen der obigen Ausführungsformen, bei denen die Transportvorrichtung eine während des Transports die Grundplatte allseitig umhüllende, sterile Umverpackung umfasst, wobei die Grundplatte eine Einrichtung zur Transportsicherung aufweist, welche seitlich von der Grundplatte abstehende grätenförmige Stege umfasst, die sich gegen Innenwände der Umverpackung verklemmen oder verspannen lassen. Die von der Grundplatte abstehende grätenförmige Stege können sich seitlich in die Innenwände einer aus geeignetem Material gefertigten Umverpackung bohren und sich dort verklemmen, verhaken oder verspannen, um ein Verrutschen der Grundplatte innerhalb der Umverpackung zu verhindern und damit einen sicheren, vor allem beschädigungsfreien Transport der Gehörknöchelchenprothese zu garantieren.

Vorzugsweise werden die grätenförmige Stege in der Ebene der Grundplatte seitlich abstehen, was insbesondere die Fertigung der Transportvorrichtung vereinfacht. Möglich sind aber auch Ausführungsformen der erfindungsgemäßen Transportvorrichtung, bei denen die grätenförmigen Stege seitlich aus der der Ebene der Grundplatte herausragen. Ebenso werden die grätenförmige Stege gegenüber einer Längsachse der Grundplatte bevorzugt abgewinkelt abstehen, wobei auch Ausführungsformen möglich sind, bei denen die Stege rechtwinklig zur Längsachse abstehen können.

Eine besonders vorteilhafte Weiterbildung in dieser Klasse von Ausführungsformen der erfindungsgemäßen Transportvorrichtung zeichnet sich dadurch aus, dass die Halterungsvorrichtung mindestens eine mit der Grundplatte über feine Stege verwindbar verbundene Einhängevorrichtung umfasst, die bei der Fertigung der Transportvorrichtung mit der Oberfläche der Grundplatte fluchtet und zum Einhängen der Gehörknöchelchenprothese gegenüber diesem flach liegenden Fertigungszustand mittels einer plastischen Verformung der Stege um etwa 90° gegenüber der Grundplatte verdreht werden kann und dann über die Oberfläche der Grundplatte herausragt.

Diese verschwenkbaren Einhängevorrichtungen können ohne größeren Fertigungsaufwand leicht so gestaltet werden, dass auch Gehörknöchelchenprothesen unterschiedlicher Größen und Geometrien sicher aufgenommen und gehaltert werden können, wobei durch die Verdrehung um ca. 90° gegenüber der Grundplatte nach dem Transport und vor der Entnahme der Prothese eine ergonomisch günstige Bearbeitungsmöglichkeit geboten wird.

Ganz besonders günstig herstellbar sind Varianten dieser Weiterbildung, bei denen die Grundplatte und die Einhängevorrichtung gemeinsam aus einem zusammen hängenden Roh-Werkstück, einem so genannten Nutzen gefertigt sind.

Die Befestigung von Gehörknöchelchenprothesen, für welche die gattungsgemäße Transportvorrichtung vorgesehen ist, wird - wie oben beschrieben - an einem Glied der Gehörknöchelchenkette während der Implantation dadurch bewirkt oder zumindest unterstützt, dass die gesamte Prothese oder wenigstens das entsprechende Befestigungselement aus einem Material mit Formgedächtnis - in der Regel die Nickel-Titan-Legierung Nitinol - hergestellt wurde, welches präoperativ einer Verformung unter definierten Temperaturbedingungen unterzogen wurde. Um nun ein beispielsweise als Schlinge ausgelegtes Befestigungselement dauerhaft zu befestigen, wird die Schlinge zunächst relativ grob um den winzigen Teilbereich des entsprechenden Gehörknöchelchens herum gelegt, genau positioniert und anschließend thermisch - meist mit einem Laser oder einer elektrischen Einrichtung - derart behandelt, dass durch gezielte Verformung des Memory-Materials ein endgültiger und - bei Körpertemperatur dann auch permanenter - Verschluss der Schlinge um das Knöchelchen herum erfolgt. Analog wird auch bei anders geformten Befestigungselementen vorgegangen.

Problematisch ist dabei jedoch, dass beim Einbringen von zu hoher Lichtleistung bzw. elektrischer Energie während der Implantation der Mittelohrprothese ungewollt eine zu große lokale Aufheizung und damit eine zu hohe Temperatur im Bereich des Befestigungselements erzeugt wird, die sich auf das Gehörknöchelchen überträgt und schnell zu einer Schädigung oder gar Nekrotisierung des empfindlichen Körpergewebes am entsprechenden Abschnitt des Gehörknöchelchens führen kann. Die ganze Operation wäre dann im Endergebnis möglicherweise sogar kontraproduktiv.

Natürlich machen die Hersteller von gattungsgemäßen Mittelohrprothesen in der Regel Angaben über optimale Verfahrensparameter bei der "Bearbeitung" ihrer Prothesen mit memory effect. Aber allein schon wegen der stark differierenden Geräte der Anwender zur thermischen Behandlung vor Ort können diese Angaben lediglich sehr breite und damit oftmals nicht sonderlich aussagekräftige Bereiche umfassen. Außerdem hilft es einem Operateur in der Praxis herzlich wenig, wenn ihm gesagt wird, welche lokale Maximaltemperatur er bei der thermischen Behandlung nicht überschreiten darf. Er weiß ohnehin, dass Eiweiß ab etwa 60°C koaguliert und Körpergewebe dauerhaft geschädigt werden kann. Vielmehr muss er eine geeignete Einstellung der Leistungsabgabe seines individuell vorhandenen Gerätes finden und außerdem auch die für seine Zwecke optimale Stelle der Energiezufuhr auf die Gehörknöchelchenprothese herausfinden, bevor er die Operation im Mittelohr durchführt.

Um dem Operateur eine unkomplizierte Möglichkeit zu eröffnen, intraoperativ kurz vor der eigentlichen Implantation der Mittelohrprothese eine für seine vorhandenen thermischen Behandlungsgeräte optimale Parametereinstellung zu ermitteln, ohne dass die Mittelohrprothese selbst auch nur berührt werden muss oder ungewünscht irgendwie verändert werden könnte, ist es vorteilhaft, wenn eine Vorrichtung zur Ermittlung einer optimierten Leistungseinstellung und/oder Energieapplikationsstelle für die Bearbeitung der intraoperativ thermisch zu behandelnden Teile der Gehörknöchelchenprothese vorgesehen ist, die eine der Prothese nachgebildete Prothesenattrappe enthält, welche zumindest in denjenigen Bereichen, in denen die zugehörige Prothese intraoperativ thermisch behandelt werden soll, mit der Gehörknöchelchenprothese hinsichtlich Material, geometrischer Gestaltung und Herstellungsverfahren identisch aufgebaut ist.

Auf diese Weise kann der Operateur zunächst die optimalen Parameter der für die Gehörknöchelchenprothese hinterher in situ vorgesehenen thermischen Behandlung im Mittelohr bereits vorher an der Prothesenattrappe mit seinen vorhandenen Geräten ermitteln und geplante Handlungsabläufe statt im Mittelohr problemlos außerhalb austesten, ohne dabei die originale Prothese auch nur berühren zu müssen und dabei eventuell ungewollt zu verformen oder gar zu beschädigen. Die so ermittelten Parameter entsprechen sehr genau den für die Original-Prothese gültigen Parametern, weil die Prothesenattrappe zumindest an den Stellen der geplanten thermischen Behandlung mit der Original-Prothese identisch übereinstimmt.

Ganz besonders bevorzugt ist vor diesem Hintergrund eine Klasse von Ausführungsformen der erfindungsgemäßen Transportvorrichtung, die sich dadurch auszeichnen, dass die Transportvorrichtung eine Halterungsvorrichtung aufweist, welche auch die oben beschriebene Prothesenattrappe verschwenkbar aufnehmen kann.

Um während des Transports nicht nur die Gehörknöchelchenprothese selbst, sondern auch die Prothesenattrappe vor thermisch bedingten Deformationen zu schützen, sind bei einer vorteilhaften Weiterbildung dieser Klasse von Ausführungsformen ein oder mehrere in die Halterungsvorrichtung einsteckbare oder in der Halterungsvorrichtung integrierte Platzhalter-Pins vorgesehen, die so geformt sind, dass sie die thermisch vorbehandelten, aus Material mit Formgedächtnis bestehenden Teile der Prothesenattrappe während des Transports zum Operateur in der gewünschten Geometrie derart fixieren, dass auch bei einer eventuellen Erwärmung während des Transports ungewünschte Formveränderungen der Prothesenattrappe verhindert werden.

Besonders bevorzugte und für den Benutzer komfortable Weiterbildungen der oben beschriebenen Ausführungsformen zeichnen sich dadurch aus, dass die Halterungsvorrichtung für die Prothesenattrappe eine mit der Grundplatte über feine Stege verwindbar verbundene weitere Einhängevorrichtung zum Einhängen eines Endes der Prothesenattrappe enthält. Damit kann nicht nur eine stabile und gut zugängliche Positionierung der Prothesenattrappe während der Ermittlung der optimalen Behandlungsparameter, sondern auch ein schonender und beschädigungsfreier Transport zum "Einsatzort" sichergestellt werden.

Eine weitere erhebliche Verbesserung der Handhabung wird bei Varianten der obigen Ausführungsformen erreicht, bei welchen die Prothesenattrappe eine Einrastvorrichtung enthält, mit welcher die Prothesenattrappe zumindest in einer Bearbeitungsposition in der Grundplatte fixierbar ist. Die starre und unverrückbare Lage der Prothesenattrappe während der Ermittlung von optimalen Parametern erleichtert nicht nur die Arbeit, sondern erhöht auch die Qualität der aufgefundenen Parameter.

Besonders bevorzugt sind auch Weiterbildungen der obigen Ausführungsformen, bei denen die Grundplatte optisch kodiert und/oder beschriftet ist, wobei die Kodierung oder Beschriftung technische Informationen bezüglich der Gehörknöchelchenprothese und/oder einer zugehörigen Prothesenattrappe und/oder Herstellerangaben enthält, so dass diese wichtigen Daten nicht erst in einem Handbuch oder Begleitzettel zur Transportvorrichtung nachgeschlagen werden müssen, sondern unmittelbar bei der Handhabung der Transportvorrichtung zur Verfügung stehen.

Von besonderem praktischen Vorteil schließlich sind Varianten dieser Weiterbildungen, bei denen die technischen Informationen der Kodierung und/oder Beschriftung einen empfohlenen Anfangswert für die elektrische Leistung oder Lichtleistung enthalten, welcher zu Beginn der Ermittlung von optimierten Bearbeitungsparametern der intraoperativ thermisch zu behandelnden Teile der Gehörknöchelchenprothese mit Hilfe einer Testbehandlung der Prothesenattrappe angewendet werden soll. Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Im Einzelnen zeigen:
- Fig. 1: eine Ausführungsform der erfindungsgemäßen Transportvorrichtung mit einer neben einer zugehörigen Gehörknöchelchenprothese auf einer Halterungsvorrichtung im Bereich einer Einrastvorrichtung verschwenkbar aufgenommenen Prothesenattrappe in einer schematischen räumlichen Darstellung von schräg oben;
- Fig. 2a: eine Detailansicht der Prothesenattrappe im Bereich der Einrastvorrichtung bei der Ausführungsform von Fig. 1;
- Fig. 2b: eine Detailansicht mit der gesamten Prothesenattrappe in einer schematischen räumlichen Darstellung von schräg oben um etwa 180° gegenüber Fig. 1 verdreht;
- Fig. 3a-3e: die Ausführungsform von Fig. 1 in schematischer Draufsicht
(a) von oben,
(b) von unten,
(c) von einer Stirnseite I,
(d) von der Gegen-Stirnseite II und
(e) von einer Längsseite III;
- Fig. 4a: die Ausführungsform von Fig. 1 in schematischer räumlicher Darstellung von schräg oben mit entnommener Gehörknöchelchenprothese und aufgerichteter und mit der Halterungsvorrichtung verrasteter Prothesenattrappe;
- Fig. 4b: wie Fig. 4a, aber um etwa 180° verdreht aus der Perspektive der gegenüber liegenden Längsseite;
- Fig. 5a: die Grundplatte der Ausführungsform von Fig. 1 ohne Gehörknöchelchenprothese und ohne Prothesenattrappe sowie mit der verschwenkbaren Halterung für die Prothese und der Einhängevorrichtung für die Attrappe jeweils im unverschwenkten, flach liegenden Fertigungs-Zustand;
- Fig. 5b: die Transportvorrichtung von Fig. 5a in einer schematischen Ansicht von unten;
- Fig. 5c: die Transportvorrichtung von Fig. 5a mit der Einhängevorrichtung für die Gehörknöchelchenprothese im verschwenkten Zustand zur Aufnahme der zu transportierenden Prothese; und
- Fig. 5d: die Transportvorrichtung von Fig. 5c in einer schematischen Ansicht von unten.

Die in den Figuren der Zeichnung schematisch dargestellte Ausführungsform der erfindungsgemäßen **Transportvorrichtung** 1 mit einer **Gehörknöchelchenprothese 2** zur Implantation ins Mittelohr, die an ihrem einen Ende ein **erstes Befestigungselement 2a** zur mechanischen Verbindung mit dem Trommelfell oder einem Glied der Gehörknöchelchenkette und an ihrem anderen Ende ein **zweites Befestigungselement 2b** zur mechanischen Verbindung mit einem weiteren Glied der Gehörknöchelchenkette oder direkt mit dem Innenohr sowie ein die beiden Befestigungselemente 2a, 2b Schall leitend miteinander verbindendes **Verbindungselement 2c** aufweist, und wobei zumindest Teile der Gehörknöchelchenprothese 2 aus einem Material mit Formgedächtnis (= memory effect) hergestellt sind, welche bei der Implantation der Gehörknöchelchenprothese 2 im Mittelohr einer Form verändernden thermischen Behandlung unterzogen werden, zeichnet sich dadurch aus, dass die Transportvorrichtung 1 eine flache **Grundplatte 5** - in der Regel aus dünnem Metallblech von geringer Dicke - umfasst, in die mindestens eine mit der Grundplatte 5 über feine **Stege 6b'** verwindbar verbundene **Einhängevorrichtung 6b** für die Aufnahme und Halterung der Gehörknöchelchenprothese 2 integriert ist, und dass die Einhängevorrichtung 6b bei der Fertigung der Transportvorrichtung 1 mit der Oberfläche der Grundplatte 5 fluchtet und zum Einhängen der Gehörknöchelchenprothese 2 gegenüber diesem flach liegenden Fertigungs-Zustand mittels einer plastischen Verformung der Stege 6b' um etwa 90° gegenüber der Grundplatte 5 verdreht werden kann und dann über die Oberfläche der Grundplatte 5 herausragt.

Die Transportvorrichtung 1 wird günstigerweise durch Bearbeitung der Grundplatte 5 mittels Laserbehandlung und/oder Anodisierung gemeinsam mit der Einhängevorrichtung 6b aus einem zusammen hängenden Nutzen hergestellt.

Des Weiteren trägt die Transportvorrichtung 1 auch eine der Gehörknöchelchenprothese 2 nachgebildete **Prothesenattrappe 3,** die Teil einer Testeinrichtung zur Ermittlung einer optimierten Leistungseinstellung und/oder Energieapplikations-Stelle für die Bearbeitung von intraoperativ thermisch zu behandelnden Teilen der Gehörknöchelchenprothese 2 ist. Diese Prothesenattrappe 3 ist zumindest in denjenigen Bereichen, in denen die zugehörige Prothese 2 intraoperativ thermisch behandelt werden soll, mit der Gehörknöchelchenprothese 2 hinsichtlich Material, geometrischer Gestaltung und Herstellungsverfahren identisch aufgebaut.

Die Gehörknöchelchenprothese 2 sowie ihre zugehörige Prothesenattrappe 3 sind bei der in Zeichnung gezeigten Ausführungsform der erfindungsgemäßen Transportvorrichtung 1 zumindest im Bereich des ersten Befestigungselements 2a aus einem Material mit Formgedächtnis hergestellt. Die Prothese kann auch eine Einrichtung zur Variation ihrer Länge aufweisen, wobei dann die Prothese sowie ihre zugehörige Prothesenattrappe zumindest im Bereich der Einrichtung zur Längenvariation aus einem Material mit Formgedächtnis hergestellt sind.

Als bevorzugtes Material mit Formgedächtnis (= memory effect) kommt bei vielen Gehörknöchelchenprothesen sowie gegebenenfalls den zugehörigen Prothesenattrappen eine Nickel-Titan-Legierung, insbesondere Nitinol zum Einsatz.

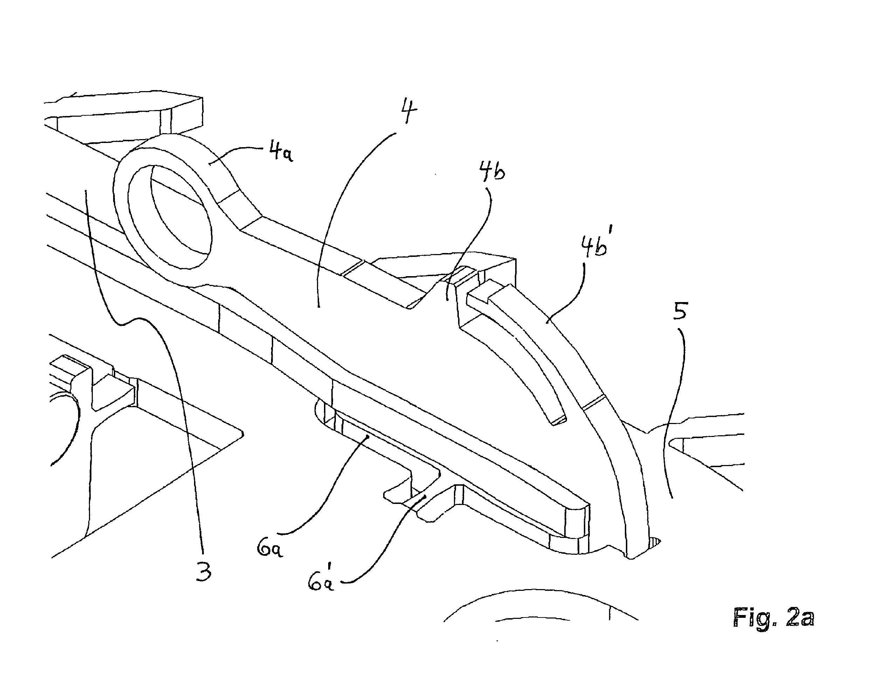

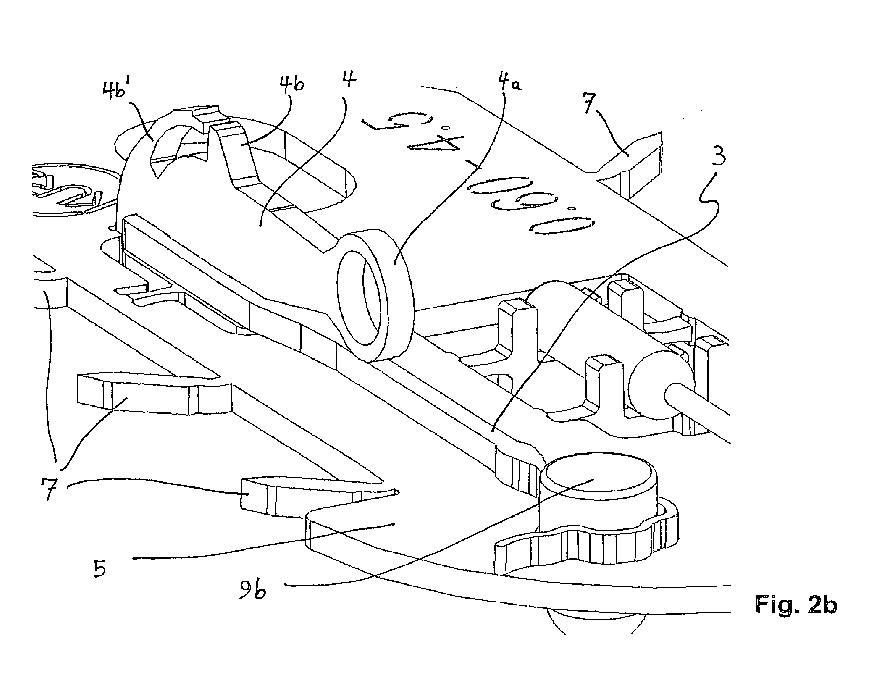

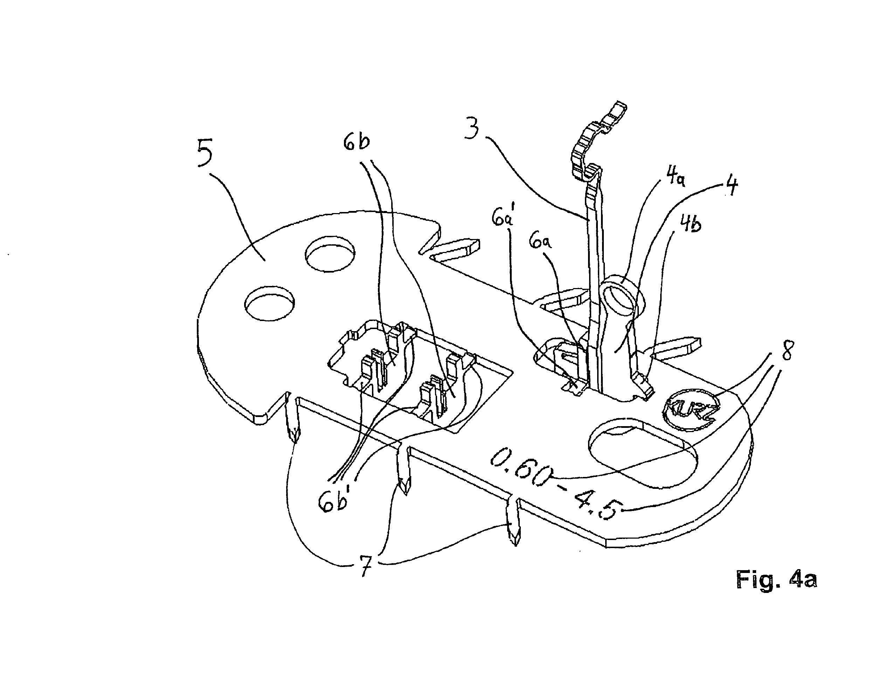

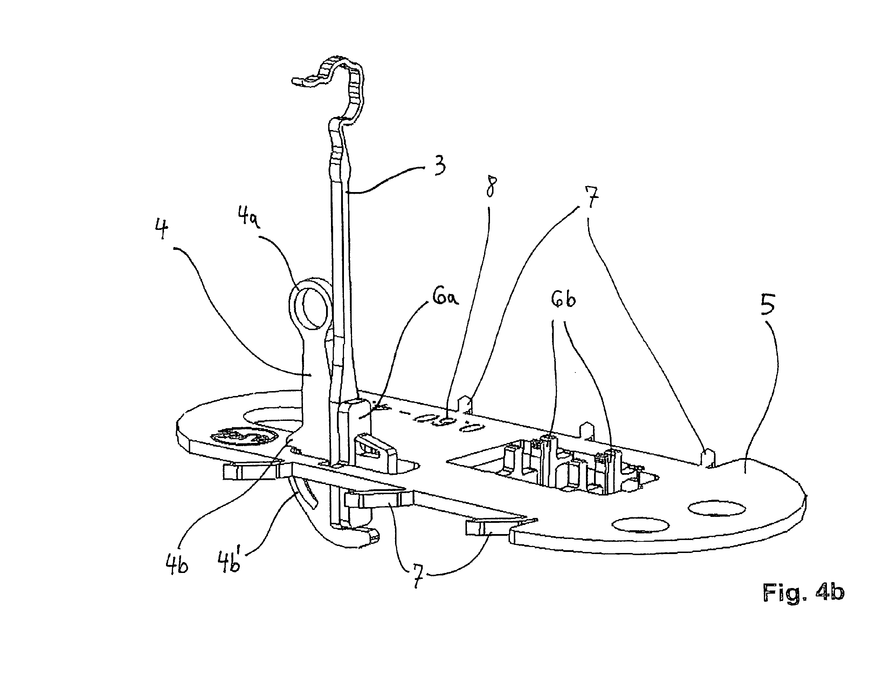

Bei allen in der Zeichnung gezeigten Darstellungen der Prothesenattrappe 3 ist eine **Einrastvorrichtung 4** vorgesehen, mit welcher die Prothesenattrappe 3 in einer - wie in den Figuren 4a und 4b zu erkennen vorzugsweise aufrechten - Bearbeitungsposition fixierbar ist. Mögliche Details der Einrastvorrichtung 4 sind in den Figuren 2a und 2b vergrößert dargestellt, etwa ein laschenförmiges **Bedienelement 4a** oder **Verriegelungselemente 4b, 4b',** die im fixierten Zustand eine Gegenhalterung umgreifen, wie in Fig. 4b gut zu erkennen ist.

Sämtliche in der Zeichnung dargestellten Ausführungsformen der Transportvorrichtung 1 stimmen auch darin überein, dass eine Halterungsvorrichtung vorgesehen ist, welche die Prothesenattrappe 3 verschwenkbar aufnehmen kann.

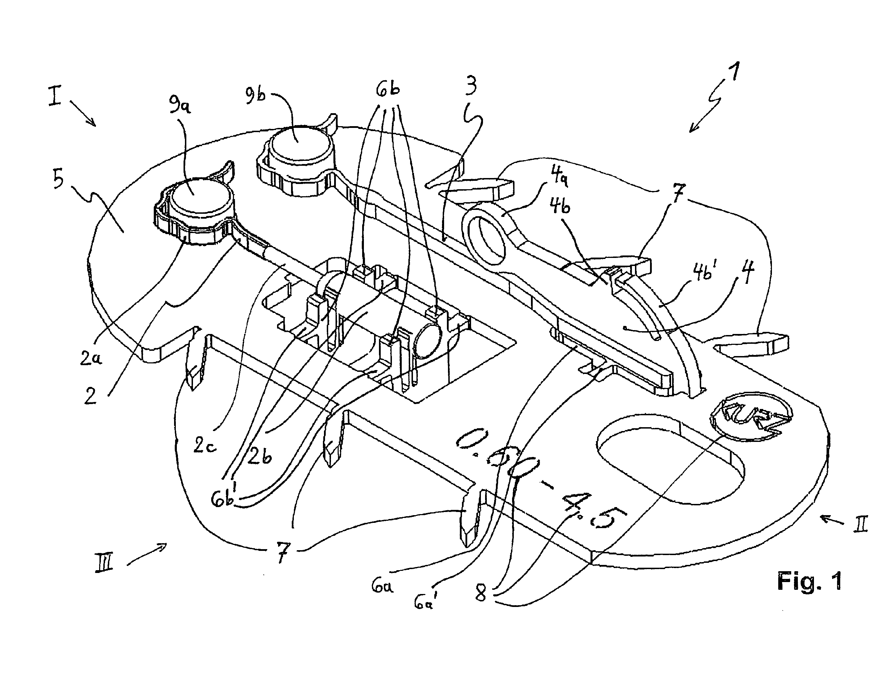

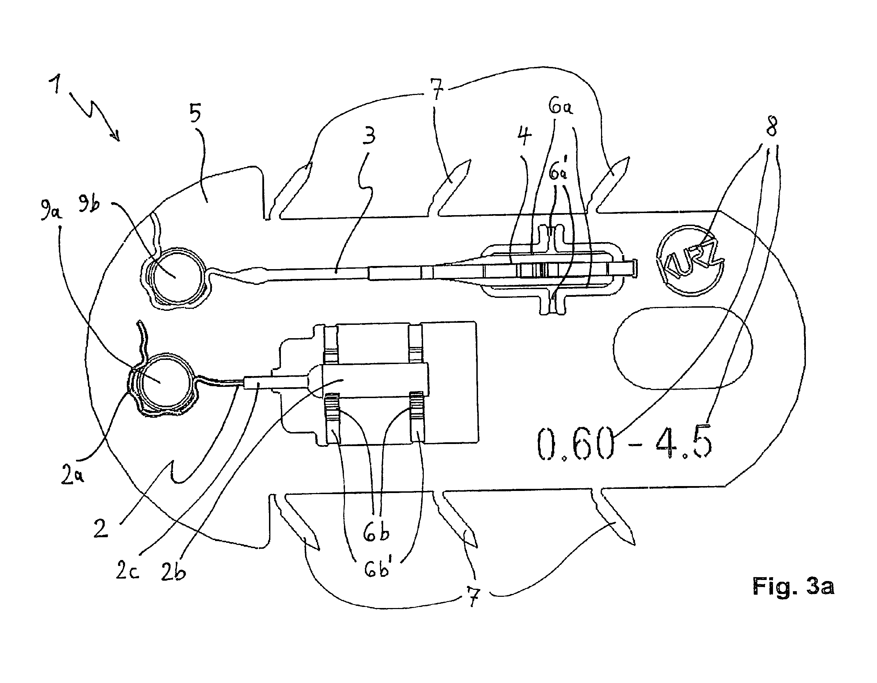

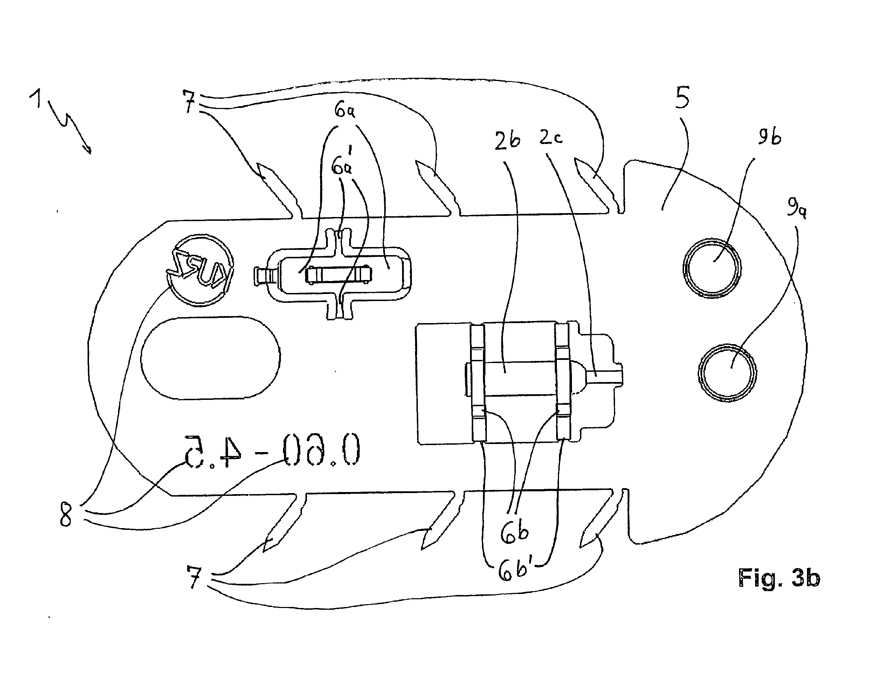

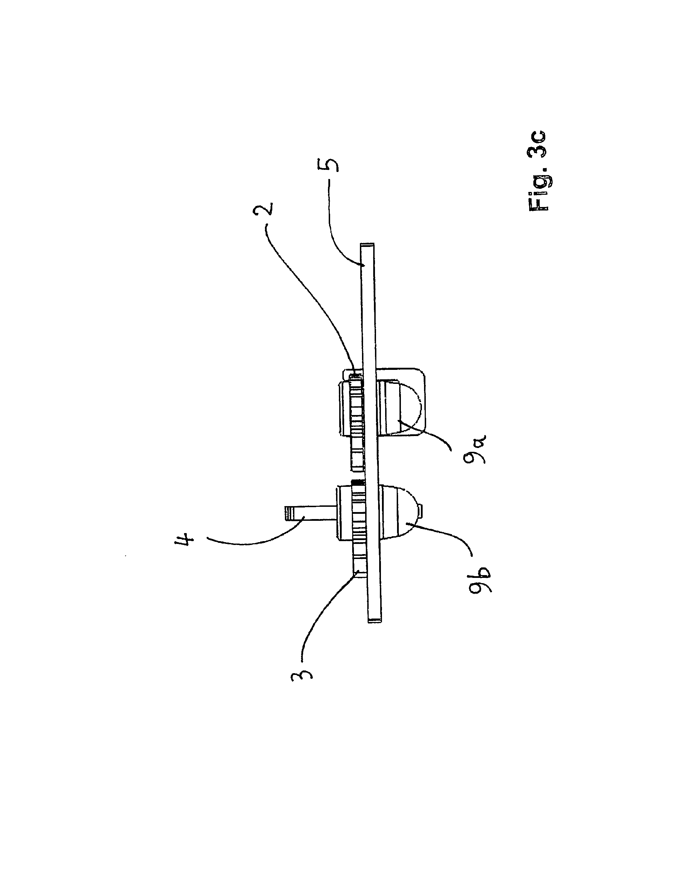

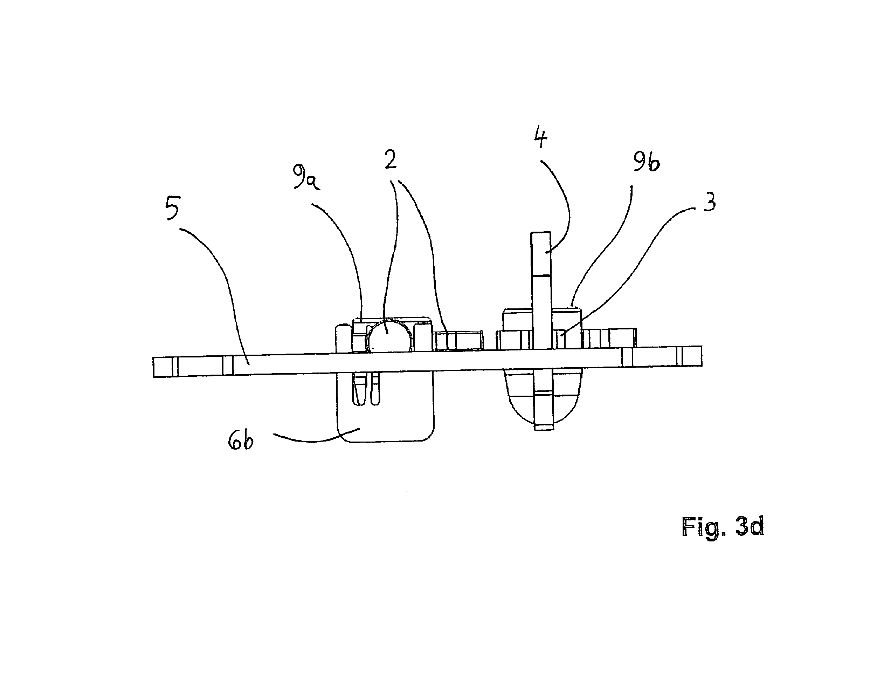

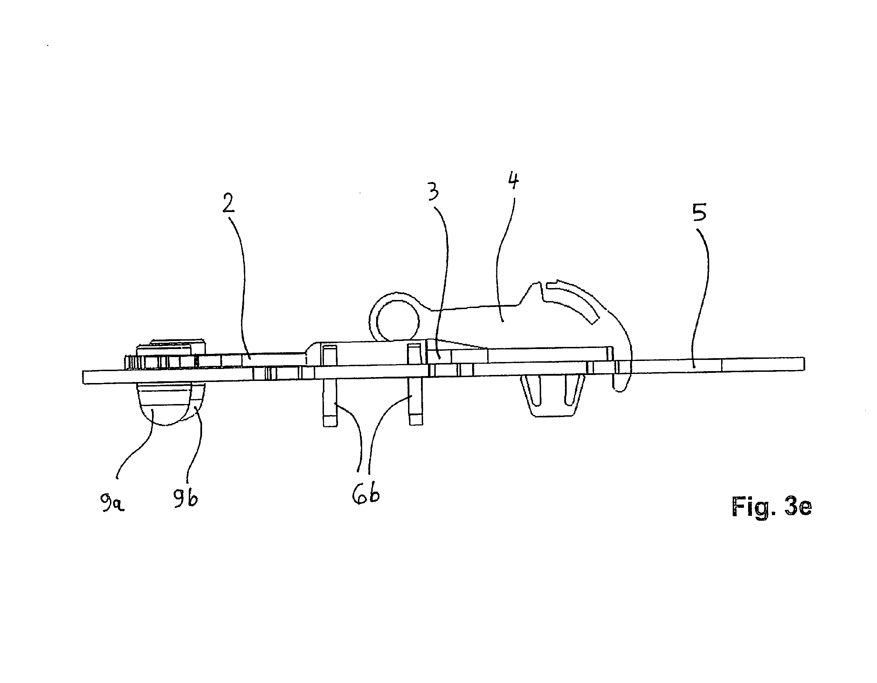

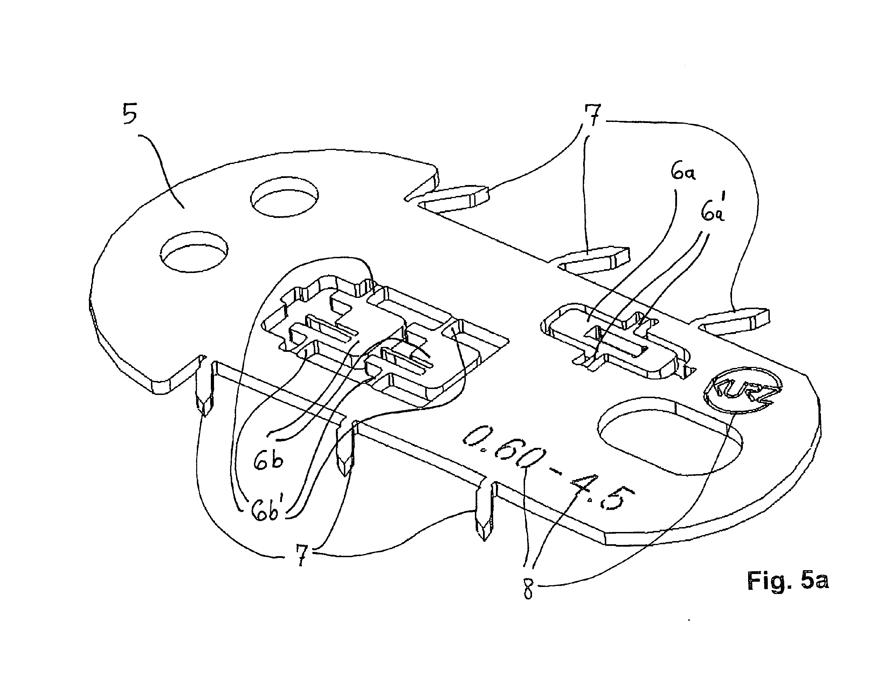

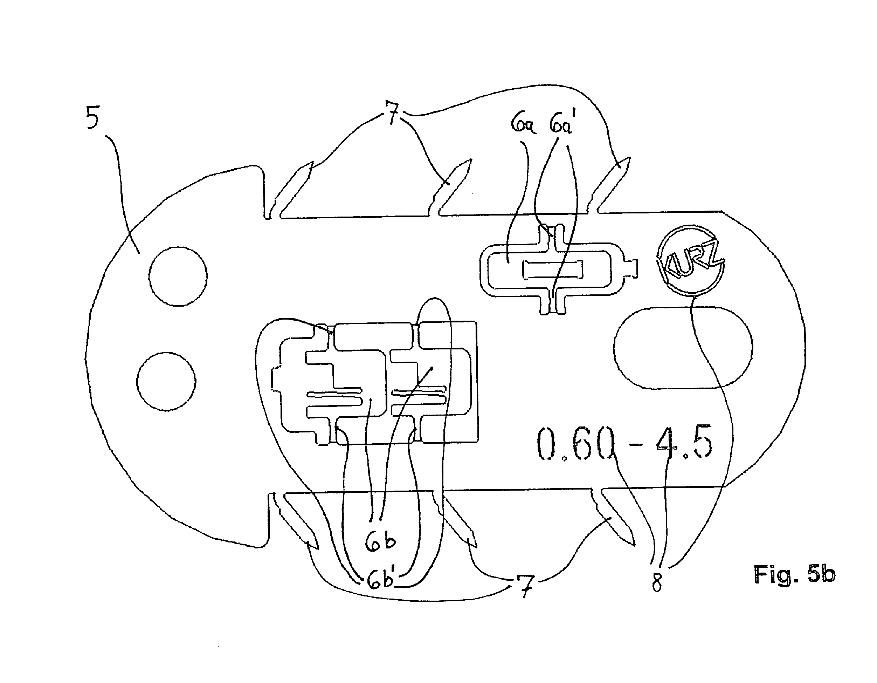

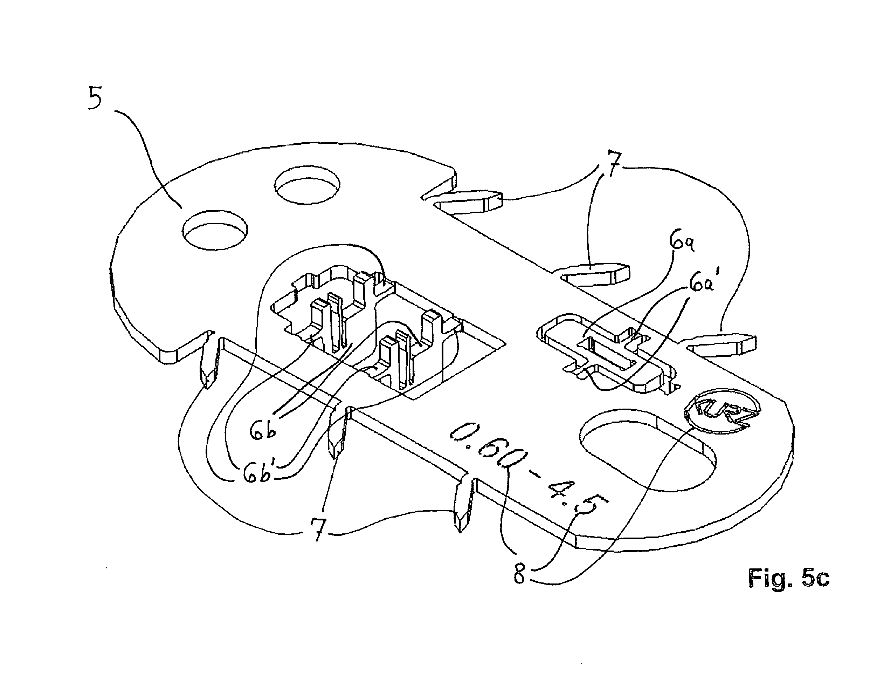

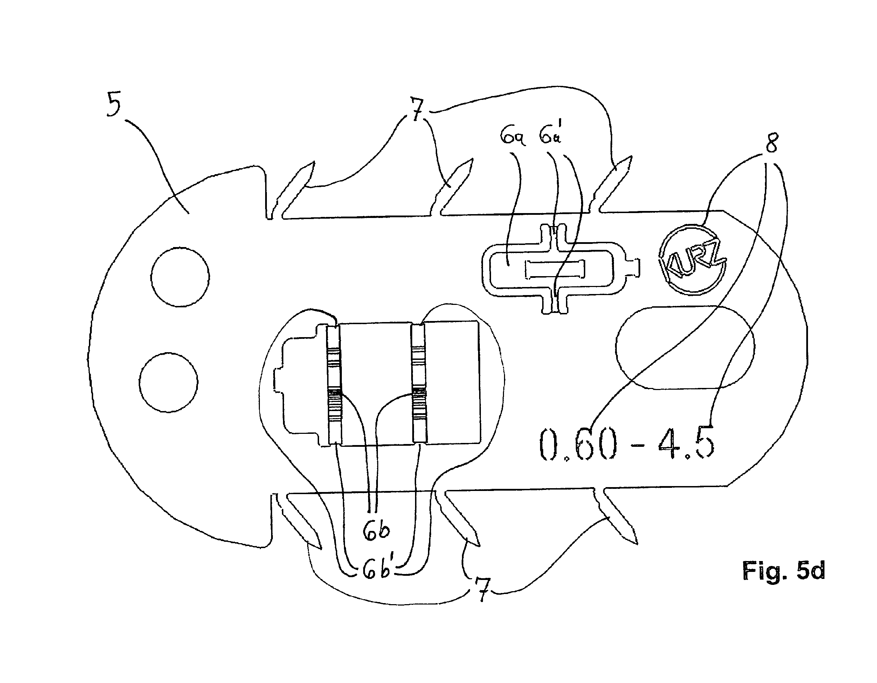

Die Figuren 1 sowie 3a bis 3e zeigen die Grundplatte 5 mit montierter Gehörknöchelchenprothese 2 und Prothesenattrappe 3 im flach liegenden Transportzustand, die Figuren 4a und 4b mit aufgestellter und verrasteter Prothesenattrappe 3 bei bereits entnommener Prothese 2 und die Figuren 5a bis 5d ohne Prothesenattrappe 3 und Prothese 2, wobei die Figuren 5a und 5b speziell den Zustand der Halterungsvorrichtung 5 unmittelbar nach deren Fertigung darstellen, während die Figuren 5c und 5d den Zustand kurz vor der Montage der Gehörknöchelchenprothese 2 auf der Halterungsvorrichtung 5 illustrieren. Nicht dargestellt sind mögliche Ausführungsformen der erfindungsgemäßen Transportvorrichtung, bei welchen die Halterungsvorrichtung derart gestaltet ist, dass sie nur die Prothesenattrappe, nicht aber die zugehörige Gehörknöchelchenprothese (oder vice versa) aufnehmen kann.

Die Figuren der Zeichnung zeigen eine besonders bevorzugte Ausführungsform der Erfindung, bei der die Halterungsvorrichtung für die Prothesenattrappe 3 eine mit der Grundplatte 5 über feine **Stege 6a'** verwindbar verbundene **Einhängevorrichtung 6a** zum Einhängen eines Endes der Prothesenattrappe 3 umfasst. Wie oben erwähnt, illustrieren Figuren 5c und 5d insbesondere den Zustand der Transportvorrichtung 1 kurz vor der Montage der Gehörknöchelchen-prothese 2, wobei die Einhängevorrichtungen 6b für die Aufnahme der Prothese 2 gegenüber dem flach liegenden Zustand in den Figuren 5a und 5b mit Hilfe der Stege 6b' um etwa 90° gegenüber der Grundplatte 5 verdreht sind. Die Prothesenattrappe 3 hingegen wird für den Transport der Testeinrichtung zum Operateur im unverdrehten Zustand der Einhängevorrichtung 6a eingehängt und erst zur Ermittlung der optimalen Bearbeitungsparameter der Gehörknöchelchenprothese 2 gegenüber der Platte um etwa 90° verschwenkt und verrastet, wie in den Figuren 4a und 4b gut zu erkennen ist.

Die Transportvorrichtung 1 wird in der Regel eine während des Transports die Grundplatte 5 allseitig umhüllende Umverpackung, insbesondere in Form eines Behälters wie einer Schachtel, einer Dose oder eines Kästchens umfassen, die in der Zeichnung nicht eigens dargestellt ist.

Des Weiteren weist die Transportvorrichtung 1 eine Einrichtung zur Transportsicherung auf, welche seitlich von der Grundplatte 5 in der Grundplattenebene abstehende **grätenförmige Stege 7** umfasst, die bei - nicht dargestellten - Ausführungsformen der Erfindung aber auch schräg aus der Grundplattenebene herausragen können. Diese Stege 7 dienen für die Transportvorrichtung 1 mit der aufmontierten, gegen mechanische Beschädigungen relativ empfindlichen Gehörknöchelchenprothese 2 und der gegebenenfalls ebenfalls aufmontierten Prothesenattrappe 3 als Verrutschsicherung während des Transports zum Operateur, wobei sich die Stege 7 gegen Innenwände der oben erwähnten Umverpackung verklemmen oder verspannen lassen.

Die Grundplatte 5 ist bei der gezeigten Ausführungsform der Erfindung optisch kodiert bzw. beschriftet. Die **Kodierung oder Beschriftung 8** enthält technische Informationen bezüglich der Gehörknöchelchen-prothese 2 und/oder der zugehörigen Prothesenattrappe 3 sowie Herstellerangaben. Die Kodierung kann auch eine einfache Farbkodierung umfassen. Alternativ oder ergänzend dazu können - bei in der Zeichnung nicht dargestellten Ausführungsformen - auch die Gehörknöchelchenprothese und/oder die zugehörige Prothesenattrappe optisch kodiert und/oder beschriftet sein, wobei die Kodierung oder Beschriftung wiederum technische Informationen über die Gehörknöchelchenprothese und/oder der Prothesenattrappe enthält.

Die technischen Informationen der Kodierung bzw. Beschriftung 8 enthalten unter anderem neben der einer Größenklassifizierung der verwendeten Gehörknöchelchenprothese 2 einen empfohlenen Anfangswert für die elektrische Leistung oder Lichtleistung, welcher zu Beginn der Ermittlung von optimierten Bearbeitungsparametern der intraoperativ thermisch zu behandelnden Teile der Gehörknöchelchenprothese 2 mit Hilfe einer Testbehandlung der Prothesenattrappe 3 angewendet werden soll.

Die Kodierung und/oder Beschriftung 8 wird am einfachsten mittels Laserbehandlung und/oder Anodisierung erzeugt. Ebenso wird die Grundplatte 5 selbst bevorzugt durch Laserschneiden der Platte aus einem größeren Blech hergestellt und weiterbearbeitet. Insbesondere die winzigen Strukturen der oben beschriebenen Elemente der Grundplatte 5, wie etwa der Einhängevorrichtungen 6a und 6b, der zugehörigen verwindbaren Stege 6a' und 6b' sowie der grätenförmige Stege 7 für die Transportsicherung lassen sich mit vertretbarem Aufwand kaum auf andere Weise herstellen.

Zum definierten Offenhalten des ersten Befestigungselements 2a der Gehörknöchelchenprothese 2 sowie des entsprechenden Abschnitts der zugehörigen Prothesenattrappe 3 während des Transports zum Operateur sind bei der erfindungsgemäßen Transportvorrichtung 1 **Platzhalter-Pins 9a, 9b** in der Grundplatte 5 vorgesehen, welche die thermisch vorbehandelten, aus Material mit Formgedächtnis bestehenden Teile der Gehörknöchelchenprothese 2 und gegebenenfalls der Prothesenattrappe 3 in der gewünschten Geometrie fixieren, so dass auch bei einer eventuellen Erwärmung während des Transports ungewünschten Formveränderungen mit Sicherheit verhindert werden können.

Die in den Figuren der Zeichnung dargestellte Ausführungsform der erfindungsgemäßen Transportvorrichtung 1 trägt eine Mittelohrprothese 2, bei welcher das erste Befestigungselement 2a die Form einer Klammer aufweist, die beispielsweise auf den Ambossfortsatz oder auch auf ein anderes Glied der Gehörknöchelchenkette aufgeklipst werden kann. Das zweite Befestigungselement 2b an dem der Klammer entgegen gesetzten Ende ist in diesem Ausführungsbeispiel als Kolben (= Piston) zur direkten Ankopplung der Gehörknöchelchenprothese 2 an das Innenohr ausgebildet.

Bei in der Zeichnung nicht dargestellten Ausführungsformen der Erfindung kann die Transportvorrichtung aber auch so ausgebildet sein, dass sie eine Gehörknöchelchenprothese aufnehmen kann, deren Befestigungselemente geometrisch anders gestaltet sind, etwa als Hülse, Schlinge oder Haken.

Das erste Befestigungselement kann eine Kopfplatte zur Anlage am Trommelfell sein. Auch kann beispielsweise das zweite Befestigungselement statt als Piston in Form einer Klammer oder stempelförmig zur Auflage auf der Steigbügelfußplatte oder als geschlitzte Glocke zur Befestigung der Gehörknöchelchenprothese auf dem Steigbügel ausgebildet sein.

In weiteren, in der Zeichnung nicht dargestellten Ausführungsformen der Erfindung kann in das Verbindungselement 2c ein Kugelgelenk integriert sein, um eine gewisse postoperative Flexibilität der Gehörknöchelchenprothese 2 zwischen ihren Verbindungsstellen sicherzustellen. Dies wird dann bei der geometrischen Ausgestaltung der Einhängevorrichtungen 6b entsprechend berücksichtigt.

Ebenfalls in der Zeichnung nicht dargestellt sind Ausführungsformen der erfindungsgemäßen Transportvorrichtung, bei denen die Grundplatte 5 als Arbeitsplatte zur Bearbeitung der Gehörknöchelchenprothese 2 vor der Implantation ins Mittelohr ausgestaltet ist. Insbesondere kann die Gehörknöchelchenprothese längenvariabel und die Grundplatte als Arbeitsplatte zur Einstellung einer individuell gewünschten Länge der Gehörknöchelchenprothese ausgestaltet sein.

Auch sind weitere mögliche Ausführungsformen nicht explizit dargestellt, bei welchen die Gehörknöchelchenprothese mit einem aktiven Vibrationsteil eines aktiven, teilweise implantierbaren Hörgeräts verbunden werden kann.

## Patentansprüche

1. Transportvorrichtung (1) mit steril verpackter Gehörknöchelchen-prothese (2) zur Implantation ins Mittelohr, wobei die Gehörknöchelchenprothese (2 mindestens teilweise die menschliche Gehörknöchelchenkette ersetzen oder überbrücken kann, und wobei zumindest Teile der Gehörknöchelchenprothese (2) aus einem Material mit Formgedächtnis (= memory effect) hergestellt sind, welche thermisch vorbehandelt und bei der Implantation der Gehörknöchelchenprothese (2) im Mittelohr einer weiteren Form verändernden thermischen Behandlung unterzogen werden, **dadurch gekennzeichnet,**
**dass** die Transportvorrichtung (1) eine Halterungsvorrichtung umfasst, die zur Aufnahme und gegen Herausfallen gesicherten Halterung der Gehörknöchelchenprothese (2) während des Transports der Prothese zum Operateur eingerichtet ist,
und **dass** ein oder mehrere in die Halterungsvorrichtung einsteckbare oder in der Halterungsvorrichtung integrierte Platzhalter-Pins (9a) vorgesehen sind, die so geformt sind, dass sie die thermisch vorbehandelten, aus Material mit Formgedächtnis bestehenden Teile der Gehörknöchelchenprothese (2) während des Transports zum Operateur in der gewünschten Geometrie derart fixieren, dass auch bei einer eventuellen Erwärmung während des Transports ungewünschte Formveränderungen der Gehörknöchelchenprothese (2) verhindert werden.

2. Transportvorrichtung nach Anspruch 1, **dadurch gekennzeichnet, dass** die Halterungsvorrichtung für die Aufnahme und Halterung der Gehörknöchelchenprothese (2) eine flache Grundplatte (5) von geringer Dicke umfasst.

3. Transportvorrichtung nach Anspruch 2, **dadurch gekennzeichnet, dass** die Grundplatte (5) aus dünnem Metallblech gefertigt ist.

4. Transportvorrichtung nach einem der Ansprüche 2 oder 3, **dadurch gekennzeichnet, dass** die Transportvorrichtung (1) durch Bearbeitung der Grundplatte (5) mittels Laserbehandlung und/oder Anodisierung hergestellt ist.

5. Transportvorrichtung nach einem der Ansprüche 2 bis 4, **dadurch gekennzeichnet, dass** die Grundplatte (5) als Arbeitsplatte zur Bearbeitung der Gehörknöchelchenprothese (2) vor der Implantation ins Mittelohr ausgestaltet ist.

6. Transportvorrichtung nach Anspruch 5, **dadurch gekennzeichnet, dass** die Gehörknöchelchenprothese (2) längenvariabel ist und die Grundplatte (5) als Arbeitsplatte zur Einstellung einer individuell gewünschten Länge der Gehörknöchelchenprothese (2) ausgestaltet ist.

7. Transportvorrichtung nach einem der Ansprüche 2 bis 6, **dadurch gekennzeichnet, dass** die Transportvorrichtung (1) eine während des Transports die Grundplatte (5) allseitig umhüllende, sterile Umverpackung umfasst, und dass die Grundplatte (5) eine Einrichtung zur Transportsicherung aufweist, welche seitlich von der Grundplatte (5) abstehende grätenförmige Stege (7) umfasst, die sich gegen Innenwände der Umverpackung verklemmen oder verspannen lassen.

8. Transportvorrichtung nach Anspruch 7, **dadurch gekennzeichnet, dass** die grätenförmige Stege (7) in der Ebene der Grundplatte (5) seitlich abstehen.

9. Transportvorrichtung nach einem der Ansprüche 2 bis 8, **dadurch gekennzeichnet, dass** die Halterungsvorrichtung mindestens eine mit der Grundplatte (5) über feine Stege (6b') verwindbar verbundene Einhängevorrichtung (6b) umfasst, die bei der Fertigung der Transportvorrichtung (1) mit der Oberfläche der Grundplatte (5) fluchtet und zum Einhängen der Gehörknöchelchenprothese (2) gegenüber diesem flach liegenden Fertigungs-Zustand mittels einer plastischen Verformung der Stege (6b') um etwa 90° gegenüber der Grundplatte (5) verdreht werden kann und dann über die Oberfläche der Grundplatte (5) herausragt.

10. Transportvorrichtung nach Anspruch 9, **dadurch gekennzeichnet, dass** die Grundplatte (5) und die Einhängevorrichtung (6b) gemeinsam aus einem zusammen hängenden Nutzen gefertigt sind.

11. Transportvorrichtung nach einem der vorhergehenden Ansprüche, **dadurch gekennzeichnet, dass** eine Vorrichtung zur Ermittlung einer optimierten Leistungseinstellung und/oder Energie-Applikationsstelle für die Bearbeitung der intraoperativ thermisch zu behandelnden Teile der Gehörknöchelchenprothese (2) vorgesehen ist, die eine der Gehörknöchelchenprothese (2) nachgebildete Prothesenattrappe (3) enthält, welche zumindest in denjenigen Bereichen, in denen die zugehörige Gehörknöchelchenprothese (2) intraoperativ thermisch behandelt werden soll, mit der Gehörknöchelchenprothese (2) hinsichtlich Material, geometrischer Gestaltung und Herstellungsverfahren identisch aufgebaut ist, und dass die Halterungsvorrichtung der Transportvorrichtung (1) so ausgebildet ist, dass sie die Prothesenattrappe (3) verschwenkbar aufnehmen kann.

12. Transportvorrichtung nach Anspruch 11, **dadurch gekennzeichnet, dass** ein oder mehrere in die Halterungsvorrichtung einsteckbare oder in der Halterungsvorrichtung integrierte Platzhalter-Pins (9b) vorgesehen sind, die so geformt sind, dass sie die thermisch vorbehandelten, aus Material mit Formgedächtnis bestehenden Teile der Prothesenattrappe (3) während des Transports zum Operateur in der gewünschten Geometrie derart fixieren, dass auch bei einer eventuellen Erwärmung während des Transports ungewünschte Formveränderungen der Prothesenattrappe (3) verhindert werden.

13. Transportvorrichtung nach einem der Ansprüche 2 bis 10 sowie nach Anspruch 11 oder 12, **dadurch gekennzeichnet, dass** die Halterungsvorrichtung für die Prothesenattrappe (3) eine mit der Grundplatte (5) über feine Stege (6a') verwindbar verbundene weitere Einhängevorrichtung (6a) zum Einhängen eines Endes der Prothesenattrappe (3) enthält.

14. Transportvorrichtung nach einem der Ansprüche 2 bis 10 sowie gegebenenfalls nach einem der Ansprüche 11 bis 13, **dadurch gekennzeichnet, dass** die Grundplatte (5) optisch kodiert und/oder beschriftet ist, und dass die Kodierung oder Beschriftung (8) technische Informationen bezüglich der
Gehörknöchelchenprothese (2) und/oder einer zugehörigen Prothesenattrappe (3) und/oder Herstellerangaben enthält.

15. Transportvorrichtung nach Anspruch 14 und einem der Ansprüche 10 bis 13, **dadurch gekennzeichnet, dass** die technischen Informationen der Kodierung und/oder Beschriftung (8) einen empfohlenen Anfangswert für die elektrische Leistung oder Lichtleistung enthalten, welcher zu Beginn der Ermittlung von optimierten Bearbeitungsparametern der intraoperativ thermisch zu behandelnden Teile der Gehörknöchelchenprothese (2) mit Hilfe einer Testbehandlung der Prothesenattrappe (3) angewendet werden soll.
